# EUROPEAN PATENT APPLICATION

(11) **EP 2 191 788 A1**
(43) Date of publication of application: **02.06.2010**
(21) Application number: 08020791.3
(22) Date of filing: 29.11.2008
(51) Int. Cl.: A61C 19/04, A61B 5/00, A61C 17/00, A61C 9/00

(54) **Method and device for three-dimensional measurement of a dental model**

(71) Applicant: Braun GmbH, 61476 Kronberg/Taunus (DE)
(72) Inventor: Bielfeldt, Uwe, 65812 Bad Soden (DE); Engelmohr, Reiner, 63329 Egelsbach (DE); Markgraf, Dirk, 61476 Kronberg/Taunus (DE); Stolper, Michael, 65760 Eschborn (DE)

(57) **Abstract**

The invention relates to a method for determining the cleaning performance of dental cleaning products. To do so a device for three-dimensional measurement of a dental model is provided. The device has two split-beam measuring devices (10,10') for generating two measurement areas of the dental model, wherein the measuring areas are different from one another. Each split-beam measuring device comprises a first (20,20') and a second (21,21') camera system and a beam of light (30,30'), wherein the optical axis (25) of the lens of the first camera system is inclined by a first triangulation angle (α₁) and the optical axis (26) of the lens of the second camera system is inclined by a second triangulation angle (α₂) with respect to the plane of the beam of light, where α₁ is different from α₂ . The dental model and the split-beam measuring devices are arranged so they can be moved in relation to one another. Two dimensions of the three-dimensional model are recorded by means of a split beam, the third dimension being generated by the relative movement. The area and layer thickness of plaque substitute residues on the three-dimensional model are determined. The cleaning performance of different dental cleaning products can thus be effectively compared.

## Description

### FIELD OF THE INVENTION

The invention relates to a method for three-dimensional measurement of a dental model. The invention also relates to a device for three-dimensional measurement of a dental model, preferably using the inventive method.

### BACKGROUND OF THE INVENTION

The determination of cleaning performance is an important parameter in the development of dental cleaning products, such as toothbrushes, dental floss, toothpaste, etc. This may be evaluated in so-called clinical tests using test subjects as well as with artificial laboratory methods. The latter have the advantage that they allow a much more rapid assessment of the cleaning performance of a novel or optimized cleaning system. To do so, dental models or dentition casts to which plaque substitutes have been applied are used. The cleaning performance is evaluated on the basis of the plaque substitutes remaining on the dental surface and/or dentition surface after a cleaning procedure.

It is known that imaging methods may be used to support this evaluation. For example, it is known that projections of the dental surfaces to be evaluated may be used as the basis for detecting residues of plaque substitutes. Projection planes here are the external and internal (buccal and lingual) dental surfaces and the chewing (occlusal) surface. One disadvantage of these methods is that surfaces and/or parts of surfaces of the teeth that are not parallel to the plane of projection, e.g., surfaces in the dental interspaces and at the root of the tooth are not reproduced in their actual size, which can falsify the result of the evaluation. This has an even greater effect on the result when the evaluation is of particular importance, especially in the interdental areas.

DE 43 01 538 A1 discloses an arrangement and a method for three-dimensional determination of a dental model. The photography method is based on the split-beam method. It is proposed here that one or more measuring heads should be used to record a three-dimensional dental model. One measuring head consists of a laser light source to generate a split beam and one photographing unit with which the split beam on the surface of the dental model is recorded. DE 43 01 538 A1 also proposes that the measuring heads should be moved around the dental model for the photograph to achieve the most complete possible photograph of the dental model. The disadvantage here is thus that, depending on the embodiment, multiple drive units are provided for positioning the measuring head(s) accordingly, which increases the structural costs and restricts the reproducibility of measurements and/or photographs and thus also has an effect on the comparability of multiple measurements in particular.

### OBJECT OF THE INVENTION

The object of the present invention is to make available a method and a device for three-dimensional measurement of a dental model which avoid the known disadvantages at least to some extent.

### INVENTIVE APPROACH

According to the present invention, this object is achieved with a method for determining the cleaning performance of dental cleaning products on a dental model, which comprises
- measurement of the dental model using plaque substitute residues as a three-dimensional model, and
- measurement of the plaque substitute residues on the three-dimensional model, whereby the area of the plaque substitute residues on the surface of the three-dimensional model is determined, and the layer thickness of the plaque substitute residues on the surface of the three-dimensional model is determined by comparing a height profile of the three-dimensional model with plaque substitute residues with a height profile of a three-dimensional model of the dental model without plaque substitute residues.

By determining the layer thickness, an even more accurate assessment can be provided, in particular when the cleaning performance of several dental cleaning products is to be compared.

Measurement of the dental models with and without plaque substitute residues as a three-dimensional model may be performed according to the split-beam method, whereby at least one beam of light which moves on the surface of the dental model is projected onto the dental model and moves on the surface thereof, whereby a first photograph of the beam of light is created at a first triangulation angle (α₁) to the projection axis of the beam of light and a second photograph of the beam of light is created at a second triangulation angle (α₂) to the projection axis of the beam of light, whereby the first triangulation angle (α₁) is different from the second triangulation angle (α₂), whereby two dimensions of the dental model are derived from the beam of light in the photographs by means of triangulation and the third dimension of the dental model is derived from the movement of the beam of light on the surface of the dental model.

It has been found to be advantageous if the movement of the beam of light on the surface of the dental model is accomplished by rotation of the dental model. It is then possible to be sure that reproducible photographs can be created, which is advantageous in particular when the cleaning performance of different dental cleaning products are to be compared with one another.

The space coordinates of the surface of the dental model are derived from the photographs and the movement of the beam of light, and a three-dimensional model for the display on a display device is generated from the space coordinates. The three-dimensional model preferably describes a height profile of the dental model (5).

The intensity distribution of the beam of light on the surface of the dental model can preferably be determined from the photographs, and a measure of the reflectivity of the surface of the dental model can be derived from this. Plaque and/or plaque substitute residues on the dental model can thus be detected in an efficient manner.

The values for the reflectivity may be assigned to a plaque substitute residue, whereby the assignment of a reflectivity value to plaque substitute residues and/or to the concentration of plaque substitute residues is made on the basis of one or more reflectivity threshold values, and whereby the plaque substitute residues and/or the concentration of plaque substitute residues may be displayed on the three-dimensional model. The surfaces of the plaque substitute residues on the three-dimensional model can be determined by means of numerical methods.

The layer thickness of the plaque substitute residues may be determined from the difference in the height profiles of the three-dimensional model with plaque substitute residues and of the three-dimensional model without residues of plaque substitute.

Furthermore, a device for three-dimensional measurement of a dental model is provided, having at least two split-beam measurement devices for generating two measurement surfaces of the dental model, whereby each of the split-beam measurement devices comprises a first camera system and a second camera system and a beam of light such that the optical axis of the lens of the first camera system is inclined by a first triangulation angle (α₁) with respect to the plane of the beam of light and the optical axis of the lens of the second camera system is inclined by a second triangulation angle (α₂) with respect to the plane of the beam of light, such that the first triangulation angle is different from the second triangulation angle, whereby the split-beam measurement devices are each arranged at a distance from the dental model, so that four different measurement surfaces of the dental model can be generated and the dental model and the split-beam measurement devices are arranged so they can be moved in relation to one another.

It is thus advantageously possible to detect the entire dental model in one recording passage, whereby undercuts on the chewing surfaces and on the side surfaces of the dental model can influence the generation of the three-dimensional dental model.

The triangulation angle (α₁) of the first camera system advantageously corresponds to the negative second triangulation angle (!α₂) of the second camera system.

The inventive device may have a rotational device for photographing the dental model such that two dimensions of the dental model can be generated by the camera systems by means of a split-beam and the third dimension of the dental model can be generated by rotation of the dental model relative to the camera systems.

The beam of light of the split-beam measurement devices can be formed by a laser light source, preferably by a semiconductor laser with collimators and microline lenses for generating the beam of light.

Measured value pickups of the camera systems may be designed so that a measure of the reflectivity of the surface of the dental model can be derived from the intensity distribution of the beam of light on the surface of the dental model.

In one embodiment of the invention, the split-beam measurement devices may be coupled to an image analyzer unit for generating a three-dimensional model of the dental model, which can be displayed on a display device, such that plaque substitute residues on the dental model can be represented on the surface of the three-dimensional model.

### BRIEF DESCRIPTION OF THE FIGURES

Additional features, possible applications and advantages of the invention are derived from the following description of exemplary embodiments of the invention, which are depicted in the figures in the drawings that form the figures:
- Fig. 1: shows a schematic partially perspective diagram of the inventive device;
- Fig. 2: shows an arrangement of the individual elements of a split-beam measurement device according to the present invention in a side view;
- Fig. 3: shows an arrangement of the individual elements of the split-beam measurement device of the invention in a view from above;
- Fig. 4: shows a tooth with plaque substitute residues; and
- Fig. 5: shows a tooth with plaque substitute residues in cross section.

### DETAILED DESCRIPTION OF THE FIGURES

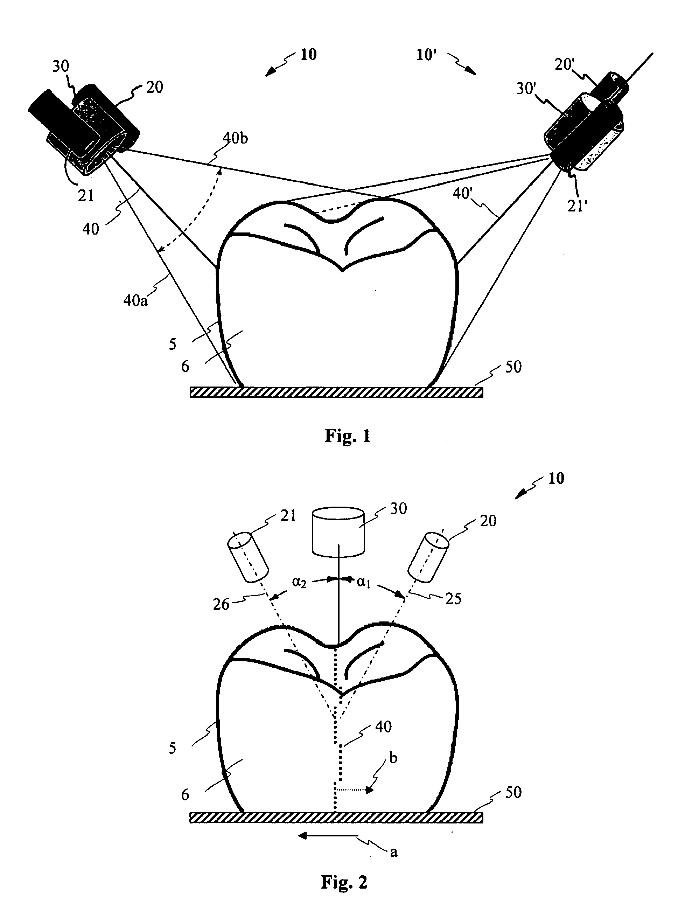

Fig. 1 shows a schematic partially perspective diagram of the inventive device for three-dimensional measurement of a dental model, but only one tooth 5 is shown here for a better overview.

Two split-beam measurement devices 10, 10', each of which has a laser light source 30, 30' and two camera systems 20, 21 and/or 20', 21' are essentially arranged on two opposing sides of the tooth 5 and/or the dental model. The split-beam measurement devices are arranged in such a way that they simultaneously image the tooth surface (buccal, lingual and occlusal) from two obliquely opposite views.

A laser beam of light 40, 40' is projected onto the tooth surfaces of the dental model by laser light sources 30, 30'. The laser beam of light is picked up by the respective camera systems 20, 21 and/or 20', 21' and analyzed by an analyzer device. The camera systems and/or the optical axes of the lenses of the camera systems are preferably arranged at a certain angle α₁ and α₂ to the laser beam of light 40, 40' and/or at a certain angle α₁ and α₂ to the plane of the surface over which the laser beam of light 40, 40' passes, as described in greater detail with respect to Fig. 2. The border of the area over which the laser beam of light 40 passes is labeled with reference numerals 40a and 40b in Fig. 1.

The tooth 5 and/or the dental model is arranged on a rotatable disk 50. By rotating the disk 50, the tooth is moved into the measurement volume of the stationary split-beam measurement devices. It is thus possible to detect an entire tooth and/or an entire dental model in three dimensions with a single recording operation. Furthermore, by adjusting the rotational speed, the resolution of the photograph can be increased or reduced in the horizontal direction.

Use of a rotating disk for fixed mounting of the dental model also has the advantage that reproducible scanning operations can be performed because the positions of the split-beam measuring device can remain fixed in space. Through position pins and/or fastenings arranged fixedly on the rotating disk, it is possible to secure a dental model on the rotating disk with accurate positioning.

Due to the opposing arrangement of the split-beam measuring devices, undercuts (as indicated by the laser beam shown with a dotted line in Fig. 1) can also be recorded because the information with respect to the concealed area can be recorded by the opposing split-beam measuring device as soon as the concealed area is moved into the measurement volume of the opposing split-beam measuring device.

In order for the camera systems of the two split-beam measuring devices 10, 10' to be able to differentiate and/or not record the beam of lights from the first laser light source 30 from the beam of lights of the other laser light source 30', the laser light sources may be operated in such a way that each generates a beam of light of a different frequency. In addition, the camera systems may be equipped with a corresponding filter, which filters out the beam of light of the other split-beam measuring device on the basis of its frequency.

A camera system may be a matrix camera. In one embodiment, telecentric lenses may be provided. In addition, special collimators and microline lenses adapted to generating the beam of light may be arranged on the camera systems.

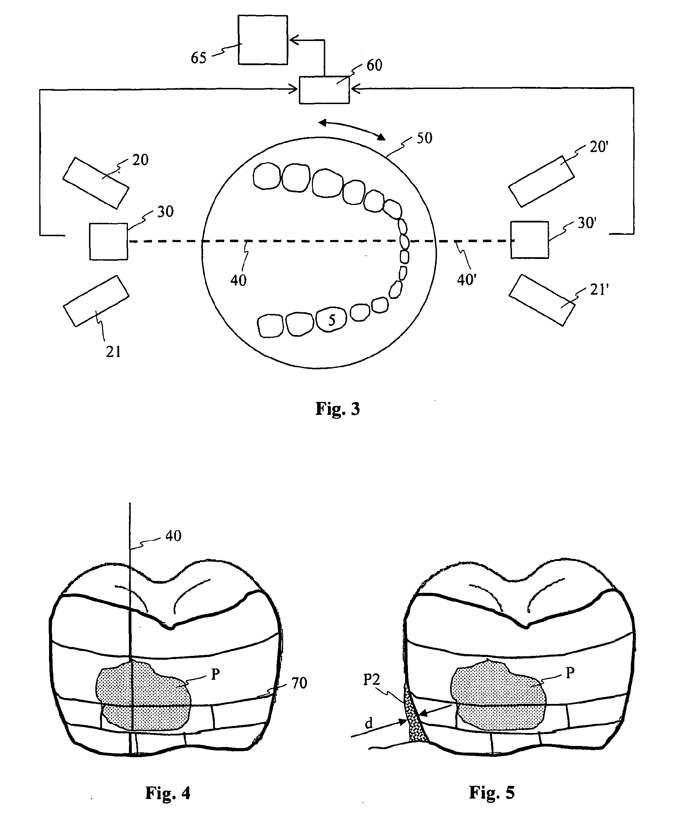

A three-dimensional model of the dentition and/or the surface of the dentition is generated from the measuring surfaces provided by the split-beam measuring devices by using a triangulation method. In addition, the surfaces of the dental model showing residues of plaque are also imaged on the three-dimensional model generated in this way (see also the description of Fig. 4). By measuring the dental model and/or the dental model twice, the thickness of the plaque substitute residues present on the dental model and/or on the dental model can also be determined (cf. description of Fig. 5).

Fig. 2 shows an arrangement of the individual elements (camera systems and laser light source) of a split-beam measuring device such as that used in the invention, shown here in a side view.

The camera systems 20, 21 and/or their lenses are inclined at a certain angle α₁ and/or α₂ relative to the laser beam of light 40, where preferably α₂ = -α₁. Due to this arrangement of two camera systems opposite the laser beam of light, it is also possible to detect and record undercuts on the side faces of the tooth. The areas that are concealed from one camera system 20 are then visible to the other camera system 21 and can be recorded.

The beam of light 40 generated by the laser light source 30 is projected onto the surface of the dental model as a beam of light and/or a split-beam. The camera systems 20, 21 detect a beam of light having a certain offset on the surface being recorded due to its inclination relative to the laser beam of light (as illustrated by the dotted-line beam of light in Fig. 2). The height information required to generate a three-dimensional model can be derived from this offset by means of known triangulation methods. The height resolution depends on the angles α₁ and/or α₂. The larger the angles α₁ and/or α₂, the greater is the height resolution because the offset of the beam of light is measurable with a greater resolution.

By rotating the disk 50 in the direction of the arrow a, the dental model arranged on the disk is also rotated in this direction. The beam of light and/or split-beam 40 imaged on the surface of the dental model moves in the direction of the arrow b. The rate of rotation enters into the measurement result together with the acquisition frequency (frequency at which the measurement surfaces are recorded by the camera systems) of the camera systems. The scanning grid therefore depends on the rate of rotation of the dental model on the rotating disk 50 and the acquisition frequency of the camera systems.

With known pixel resolution of the camera systems, the rate of rotation of the rotating disk may be adapted in such a way that preferably a sampling grid with the same resolution in width and height is achieved.

Fig. 3 shows an arrangement of the individual elements (camera systems and laser light source) of the split-beam measuring devices according to the invention in a view as seen from above. To illustrate the method, a mandible 5 is arranged here on the rotating disk 50.

A split-beam measuring device is arranged on both sides of the mandible. The split-beam measuring device arranged on the left side of the mandible records the interior (lingual) surface of the dentition in the position of the mandible illustrated in Fig. 3, while at the same time the split-beam measuring device arranged on the right side of the mandible records the exterior (buccal) surface of the dentition. In an arrangement of the split-beam measuring devices obliquely above the dental model (as shown in Fig. 1), the chewing (occlusal) surfaces are recorded by the two split-beam measuring devices.

The split-beam measuring devices are coupled to an image analyzer unit 60. The image analyzer unit 60 generates a three-dimensional model of the dental model 5 by means of triangulation methods from the measurement surfaces recorded. The three-dimensional model may then be displayed as needed on a display device 65 coupled to the image analyzer unit 60, e.g., on display screen. In addition, the three-dimensional model may also be stored for further processing. Likewise, the measurement surfaces recorded may be saved to allow analysis, e.g., generation of a 3D model, at a later point in time.

Fig. 4 shows a three-dimensional model of a tooth from the front (buccally) generated with the help of the inventive device. An area P in which residues of a plaque substitute substance are to be found on the dental model after a cleaning procedure are visibly displayed on the dental surface. The information for the display of plaque substitute residues on the dental surface is obtained according to this invention by the fact that the split-beam measuring devices and/or an image analyzer unit coupled to the split-beam measuring devices determines the intensity distribution of the beam of light 40 on the dental surface. The intensity distribution of the beam of light is different in the area of the plaque substitute residue from the intensity distribution on the clean tooth surface.

A measure of the reflectivity of the surface of the tooth and/or of the dental model is derived from the intensity distribution of the beam of light 40. Taking into account one or more reflectivity threshold values, the distribution and/or concentration of plaque substitute residues on the dental surface is determined and displayed on the three-dimensional dental model. With the help of the reflectivity threshold values, a decision is made about whether or not these are plaque substitute residues. The reflectivity threshold values are preferably adjustable. The areas of the plaque substitute residues on the surface of the tooth are ascertained by means of numerical methods.

For better comparability of different cleaning procedures, e.g., with the help of different tooth cleaning products on a dental model, the surface of the teeth is divided into cells 70, also known as grids. The number and distribution of the cells on the surface of the tooth may depend on various known plaque indices such as the Quigley-Hein index. The classification of the dental surface in the corresponding cells is performed manually or automatically by the image analyzer unit 60. The cell area may be determined by numerical methods.

The degree of plaque substitute residues in the respective cell is assigned to each cell. The degree of plaque substitute residues may be expressed as the percentage of the cell area or as the absolute area, e.g., in mm². The allocation is preferably made automatically. The comparison of various cleaning procedures on a dental model may be performed then on the basis of the degree of plaque substitute residues in the respective cells. The absolute area of plaque substitute residues on the whole or within a cell can be determined by means of known numerical methods.

Preferably the three-dimensional model together with the cells and the degree of plaque substitute residues can be saved, so that cleaning procedures at different points in time can be compared with one another.

To further improve the relevance of the measurement results with regard to the cleaning performance, the inventive method proposes determining the layer thickness of the plaque substitute residues remaining on the dental surface of the dental model after a cleaning procedure. Thus together with the degree of plaque substitute residues in a cell, an even more differentiated comparison of the cleaning performance of different dental cleaning products may be obtained.

Fig. 5 shows a three-dimensional model of a tooth generated with the help of the inventive device as seen from the front (buccally) with plaque substitute residues P and P2 in the interdental area and/or on the front (buccal) side of the tooth, where the plaque substitute residues P2 are shown in cross section to illustrate a layer thickness measurement of plaque substitute residues.

To measure the layer thickness of plaque substitute residues on the dental surface, the height information on the dental surface and/or the dentition surface as determined by means of the split-beam method and/or the triangulation method is used. To do so, in a first step a three-dimensional model of a tooth model or a dental model without plaque substitute residues is generated. This three-dimensional model serves as a reference model for the determination of the thickness of plaque substitute residues, i.e., residues of plaque substitutes.

The dental model and/or the tooth model is provided with plaque substitute residues and is then subjected to a cleaning procedure.

In another step, a second three-dimensional model is generated for the cleaned tooth model and/or dental model. By comparing the height information of the reference model with the second three-dimensional model, the thickness of the plaque substitute residues remaining on the surface of the tooth model and/or dental model is ascertained. The height information differs essentially at the locations where the plaque substitute residues are found. The thickness of the plaque substitute residues is then calculated by forming the difference in the height information.

Fig. 5 shows plaque substitute residues P2 in the interdental area of a tooth. The difference in the surface area of the tooth from the surface area of the plaque substitute residue P2 forms the thickness d of the plaque substitute residue P2.

To avoid measurements of difference for dental areas where there are no plaque substitute residues and/or rule them out, it is possible to provide for a difference measurement to be performed only with regard to the area of the tooth surface where plaque substitute residues are in fact located. These relevant areas may be ascertained with the method already described above on the basis of the intensity measurement of the beam of light. This has the advantage that differences in height information which may occur, e.g., due to nonidentical arrangement of the dental model on the rotating disk 50, cannot be detected as the layer thickness of a plaque substitute residue.

The layer thickness d of the plaque substitute residues may be stored together with the degree of the plaque substitute residues in a cell, for example. It is thus possible to compare the cleaning performance of various dental care products with regard to the thickness of the remaining plaque substitute residues.

### List of Reference Numerals

| | | |
|---|---|---|
| a | | direction of rotation of the rotating disk |
| b | | movement of the beam of light |
| P, P2 | area with plaque substitute residues | |
| α₁, α₂ | angle of inclination of the camera units | |
| 5 | | tooth model and/or dental model |
| 6 | | surface of the tooth model and/or dental model |
| 10, 10' | split-beam measurement devices | |
| 20, 21, 20', 21' | camera systems | |
| 20, 25 | optical axis of the camera systems | |
| 30, 30' | laser light source | |
| 40, 40' | beam of light and/or split beam | |
| 40a, 40b | plane of the area covered by beam of light 40 | |
| 50 | | rotating disk |
| 60 | | image analyzing unit |
| 65 | | display device |
| 70 | | grid of a plaque index |

## Claims

1. A method for determining the cleaning performance of dental cleaning products on a dental model comprising:
- detecting the dental model (5) with plaque substitute residues (P, P2) as a three-dimensional model, and
- ascertaining the plaque substitute residues (P, P2) on the three-dimensional model, whereby the area of the plaque substitute residues (P, P2) on the surface of the three-dimensional model is determined and whereby the layer thickness (d) of the plaque substitute residues (P, P2) on the surface of the three-dimensional model is determined by comparing a height profile of the three-dimensional model with plaque substitute residues (P, P2) with a height profile of a three-dimensional model of the dental model (5) without plaque substitute residues.

2. The method according to Claim 1, wherein the dental model (5) is detected with and without plaque substitute residues as a three-dimensional model according to the split-beam method; wherein at least one beam of light (40, 40') is projected onto the dental model (5) and moves on the surface (6) of the dental model (5); wherein a first photograph of the beam of light (40, 40') is generated at a first triangulation angle (α₁) to the projection axis of the beam of light (40, 40') and a second photograph of the beam of light (40, 40') is generated; wherein the first triangulation angle (α₁) is different from the second triangulation angle (α₂); wherein two dimensions of the dental model (5) are derived by triangulation from the beam of light (40, 40') in the photographs; and the third dimension of the dental model (5) being derived from the movement of the beam of light (40, 40') on the surface (6) of the dental model (5).

3. The method according to Claim 2, wherein the movement of the beam of light (40, 40') takes place on the surface (6) of the dental model (5) by rotation of the dental model (5).

4. The method according to any one of Claims 2 or 3, wherein the space coordinates of the surface (6) of the dental model (5) are derived from the photographs and the movement of the beam of light (40, 40'), and a three-dimensional model for a display on a display device (65) is generated from the space coordinates, such that the three-dimensional model describes a height profile of the dental model (5).

5. The method according to any one of Claims 2 to 4, wherein the intensity distribution of the beam of light (40, 40') on the surface (6) of the dental model (5) is determined from the photographs and a measure of the reflectivity of the surface (6) of the dental model is derived therefrom.

6. The method as recited in Claim 5, wherein values for the reflectivity of the surface (6) are assigned to a plaque substitute residue, the assignment of a reflectivity value to plaque substitute residues being made on the basis of at least one reflectivity threshold value.

7. The method according to Claim 6, wherein the plaque substitute residues are displayed on the three-dimensional model and the surfaces of the plaque substitute residues on the three-dimensional model are determined by means of numerical methods.

8. The method according to any one of Claims 4 to 7, wherein the layer thickness (d) of the plaque substitute residues (P, P2) is determined from the difference in the height profiles of the three-dimensional model with plaque substitute residues (P, P2) and the three-dimensional model without plaque substitute residues (P, P2).

9. A device for three-dimensional measurement of a dental model, comprising at least two split-beam measuring devices (10, 10') for generating two measuring surfaces of the dental model (5),
wherein each of the split-beam measuring devices (10, 10') comprises a first camera system (20, 20') and a second camera system (21, 21') and a beam of light (30, 30');
wherein the optical axis (25) of the first camera system (20, 20') is inclined by a first triangulation angle (α₁) with respect to the plane (40a, 40b) of the beam of light (30, 30');
wherein the optical axis (26) of the second camera system (21, 21') is inclined by a second triangulation angle (α₂) with respect to the plane (40a, 40b) of the beam of light (30, 30');
wherein the first triangulation angle (α₁) is different from the second triangulation angle (α₂);
wherein the split-beam measuring devices (10, 10') are each arranged at a distance from the dental model (5), so that four different measuring surfaces of the dental model (5) can be generated, and
wherein the dental model (5) and the split-beam measuring devices (10, 10') are arranged so they are movable relative to one another.

10. The device according to Claim 9, wherein the first triangulation angle (α₁) of the first camera system (20, 20') corresponds to the negative second triangulation angle (!α₂) of the second camera system (21, 21').

11. The device according to any one of Claims 9 through 10, additionally having a rotating device (50) to hold the dental model (5), wherein two dimensions of the dental model (5) can be generated by the camera systems (20, 20', 21, 21') by means of a beam of light (40, 40') and the third dimension of the dental model (5) can be generated by rotation of the dental model (5) relative to the camera systems (20, 20'; 21, 21').

12. The device according to any one of Claims 9 to 11, wherein the camera systems (20, 20'; 21, 21') have telecentric lenses.

13. The device according to any one of Claims 9 to 12, wherein the beam of lights (30, 30') of the split-beam measuring devices (10, 10') are formed by a laser light source, preferably a semiconductor laser having collimators and microline lenses adapted to generate the beams of light (40, 40').

14. The device according to any one of Claims 9 to 13, wherein the measured value pickup of the camera systems (20, 20'; 21, 21') are designed so that a measure of the reflectivity of the surface (6) of the dental model (5) can be derived from the intensity distribution of the beams of light (4, 4') on the surface (6) of the dental model (5).

15. The device according to any one of Claims 9 to 14, additionally having an image analyzing unit (60) that can be connected to the split-beam measuring devices (10, 10') for generating a three-dimensional model of the dental model (5) that can be displayed on a display device (65), wherein plaque substitute residues (P) on the dental model (5) can be displayed on the three-dimensional model and their area can be determined by means of numerical methods.
